(19) Europäisches Patentamt
European Patent Office
Office européen des brevets

(11) **EP 2 826 524 B1**

(12) **EUROPÄISCHE PATENTSCHRIFT**

(45) Veröffentlichungstag und Bekanntmachung des Hinweises auf die Patenterteilung:
**22.07.2015 Patentblatt 2015/30**

(51) Int Cl.:
***A61N 1/365*** *(2006.01)*   ***A61N 1/368*** *(2006.01)*
***A61N 1/37*** *(2006.01)*

(21) Anmeldenummer: **14168458.9**

(22) Anmeldetag: **15.05.2014**

(54) **Herztherapiegerät zum Erfassen ventrikulärer Tachykardien und Fibrillationen**

Heart therapy device for detecting ventricular tachycardias and fibrillations

Appareil de traitement cardiaque destiné à détecter des tachycardies ou fibrillations ventriculaires

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.07.2013 US 201361847093 P**
**14.08.2013 US 201361865625 P**

(43) Veröffentlichungstag der Anmeldung:
**21.01.2015 Patentblatt 2015/04**

(73) Patentinhaber: **BIOTRONIK SE & Co. KG**
**12359 Berlin (DE)**

(72) Erfinder:
• **Dörr, Thomas**
**12437 Berlin (DE)**
• **Kucher, Andreas**
**16303 Schwedt (DE)**

(74) Vertreter: **Lindner-Vogt, Karin L. et al**
**Biotronik SE & Co. KG**
**Corporate Intellectual Properties**
**Woermannkehre 1**
**12359 Berlin (DE)**

(56) Entgegenhaltungen:
**US-A1- 2007 255 327    US-A1- 2009 125 077**
**US-A1- 2011 082 512    US-A1- 2013 030 314**

EP 2 826 524 B1

**Beschreibung**

Die Erfindung betrifft ein implantierbares biventrikuläres Herztherapiegerät mit einer Therapiegerätsteuerung, die eine Tachykardie-Erkennungseinheit aufweist, welche wenigstens indirekt mit einer rechtsventrikulären Wahrnehmungselektrode und einer linksventrikulären Wahrnehmungselektrode derart verbunden oder zu verbinden ist, dass der Tachykardie-Erkennungseinheit im Betrieb einen zeitlichen Verlauf elektrischer Potentiale im Herzen repräsentierende Signale oder daraus abgeleitete Signale zugeführt werden. Die Tachykardie-Erkennungseinheit ist ausgebildet, das ihr zugeführte Signal bzw. dessen zeitlichem Verlauf auszuwerten und ein Tachyarrhythmiesignal zu generieren, falls das zugeführte Signal vorgegebene Kriterien, z.B. Frequenzkriterien hinsichtlich bestimmter Signalmerkmale wie detektierter R-Wellen, erfüllt. Durch die Ausgabe eines Tachyarrhythmiesignals signalsiert die Tachykardie-Erkennungseinheit somit eine (pathologische) Tachykardie oder Fibrillation. Das Herztherapiegerät ist typischerweise ein implantierbarer Kardioverter/Defibrillator (ICD).

Die Tachykardie-Erkennungseinheit erlaubt somit eine biventrikuläre Detektion von Tachyarrhythmien wie ventrikulären Tacchykardien (VT) oder Fibrillationen (VF). Die Therapiegerätsteuerung kann daraufhin adäquate Therapien auslösen.

Ein als S-ICD-System bekanntes System arbeitet mit einem Far-Field-Kanal zur VT/VF-Erkennung.

Eine besondere Art von Tachyarrhythmien sind "dissimilare" ventrikuläre Tachykardien, bei denen im rechten Ventrikel (RV) und im linken Ventrikel (LV) unterschiedliche (Schlag- oder Kontraktions-) Frequenzen herrschen.

Die Erfinder haben erkannt, dass derzeitige biventrikuläre Herztherapiegeräte in Bezug auf dissimilare ventrikuläre Tachykardien unzulänglich sind. Ziel der Erfindung ist es, ein biventrikuläres Herztherapiegerät zu schaffen, welches auf dissimilare ventrikuläre Tachykardien adäquat reagieren kann.

Erfindungsgemäß wird diese Aufgabe durch ein implantierbares biventrikuläres Herztherapiegerät der eingangs genannten Art gelöst, bei dem die Tachykardie-Erkennungseinheit ausgebildet ist, für die Erkennung von ventrikulären Tachykardien simultan die Herzfrequenz an der rechtsventrikulären und an der linksventrikulären Elektrode zu bewerten.

Außerdem weist die Therapiegerätsteuerung eine Dislokations-Erkennungseinheit auf, die ebenfalls wenigstens indirekt mit einer rechtsventrikulären Wahrnehmungselektrode und einer linksventrikulären Wahrnehmungselektrode derart verbunden oder zu verbinden ist, dass der Dislokations-Erkennungseinheit im Betrieb einen zeitlichen Verlauf elektrischer Potentiale im Herzen repräsentierende Signale oder daraus abgeleitete Signale zugeführt werden. Die Dislokations-Erkennungseinheit ist ausgebildet, simultan die Herzfrequenz an der rechtsventrikulären und der linksventrikulären Elektrode zu bewerten und immer dann eine rechts- bzw. linksventrikuläre Dislokation zu signalisieren und ein entsprechendes Dislokationssignal zu generieren, wenn die Dislokations-Erkennungseinheit einen plötzlichen Frequenzanstieg an der rechts- bzw. linksventrikulären Elektrode wahrnimmt ohne dass innerhalb eines vorgegebenen und/oder einstellbaren Zeitfensters eine nennenswerte Rhythmusänderung an der links- oder der rechtventrikulären Elektrode erfasst wird. Die Therapiegerätsteuerung ist ausgebildet, bei einer signalisierten Dislokation der rechtsventrikulären oder der linksventrikulären Elektrode die Rhythmusinformationen der betroffenen Elektrode für die Tachykardiedetektion zu ignorieren.

Die der Tachykardie-Erkennungseinheit und der Dislokations-Erkennungseinheit zugeführten, links- und/oder rechtsventrikulären Signale können aus den von den jeweiligen Elek-troden wahrgenommenen Signalen abgeleitete Signale sein, z.B. an sich bekannte Markersignale, die von entsprechenden rechts- linksventrikulären Sensingeinheiten erzeugt werden, wenn sie rechtsventrikuläre oder eine linksventrikuläre Kammerkontraktion z.B. anhand einer entsprechenden R-zacke im Elektrokardiogramm detektieren.

Das erfindungsgemäße Herztherapiegerät kann eine adäquate antitachykarde Therapie für die ICD-Patienten sicherstellen, bei denen dissimilare ventrikuläre VT/VF-Episoden auftreten, d.h. tachykarde Rhythmusstörungen, die im rechten Ventrikel und im linken Ventrikel unterschiedlich schnell verlaufen. Gleichzeitig kann das erfindungsgemäße Herztherapiegerät vermeiden, dass über eine dislozierte Ventrikelelektrode ein Vorhofflimmern versehentlich in die VT/VF-Detektion einfließt.

Ein derartiges Herztherapiegerät erlaubt es außerdem, bei Patienten mit unterschiedlich schnellen ventrikulären Tachykardien in beiden Ventrikeln rechtzeitig eine adäquate Therapie abzugeben, ohne dass bei Vorliegen einer Elektrodendislokation eine inadäquate Therapie abgegeben wird. Ein Vorteil des erfindungsgemäßen Herztherapiegerätes ist somit auch die Vermeidung von Fehldetektionen bei einer dislozierten Sonde und gleichzeitigem Vorhofflimmern.

Die Erfindung schließt die Erkenntnis ein, dass bisherige marktverfiigbare ICD-Systeme ausschließlich mit einem rechtsventrikulären VT/VF-Erkennungskanal arbeiten. Die linksventrikulären Sensingsignale werden hier nur zur Inhibierung nicht notwendiger LV-Stimulation und zur Aufzeichnung eines intrakardialen Elektrokardiogramms (IEGM) verwendet, nicht aber zur VT/VF-Erkennung. Ausgehend von der Beobachtung, dass es ventrikuläre Tachykardien gibt, die einen erheblichen Frequenzunterschied zwischen rechtem und linkem Ventrikel über eine nennenswerten Zeitraum aufweisen, muss davon ausgegangen werden, dass bei einer nur rechtsventrikulären Detektion eine potentielle Unterversorgung von Patienten mit diesen Rhythmusstörungen auftritt.

Insbesondere bei einem sehr viel schnelleren VT/VF im linken Ventrikel bei einer moderaten VT im rechten Ventrikel (mehrfach beobachtet) besteht die Gefahr eines letalen Ausgangs der Rhythmusstörung, da die Zeit bis zu einer effektiven Defibrillation durch die Unterschätzung mittels allein rechtsventrikulärer Wahrnehmung deutlich zu lang wird. Die Erfinder haben auch erkannt, dass eine rein biventrikuläre Wahrnehmung, wie sie in Anbetracht des Standes der Technik naheliegend wäre, die Gefahr birgt, dass z.B. bei einer in dem Bereich des Atriums dislozierten linksventrikulären Elektrode (Coronar Sinus Elektrode) ein Vorhofflimmern als linksventrikuläres Kammerflimmern (linksventrikuläre VF) fehlklassifiziert wird und so zu einer inadäquaten Therapieabgabe führt. Eine dislozierte rechtsventrikuläre Elektrode kann einen vergleichbaren Effekt hervorrufen.

Als besonders relevant sind in diesem Zusammenhang isolierte linksventrikuläre Tachyarrythmien zu nennen, da diese bei den bestehenden rechtventrikulären Systemen nicht korrekt detektiert und therapiert werden. Die Analyse biventrikulärer IEGM-Aufzeichnungen aktueller CRT-D-System zeigt jedoch einen nennenswerten Anteil derartiger Rhythmusstörungen.

Das erfindungsgemäße Herztherapiegerät ergibt im Betrieb einen implantierbaren Defibrillator mit mindestens einer rechtsventrikulären Elektrode und mindestens einer linksventrikulären (bevorzugt Coronar Sinus) Elektrode, die jeweils mit einer Tachykardie-Erkennungseinheit wirkverbunden sind, wobei die Tachykardie-Erkennungseinheit so ausgeführt ist, dass sie für die Erkennung von ventrikulären Tachykardien simultan die Herzfrequenz an der rechtsventrikulären und an der linksventrikulären Elektrode bewertet. Mit rechtsventrikuläre bzw. linksventrikuläre Elektrode ist hier jeweils ein entsprechender Wahrnehmungselektrodenpol zur bipolaren oder unipolaren Wahrnehmung von elektrischen Potentialverläufen im Myokard der jeweiligen Herzkammer gemeint, der z.B. Teil einer entsprechenden Elektrodenleitung ist und über diese mit dem Herztherapiegerät verbunden ist. Die zusätzliche Dislokations-Erkennungseinheit erlaubt das Detektieren einer möglichen Dislokation einer der ventrikulären Elektroden und ist ausgebildet, simultan die Herzfrequenz an der rechtsventrikulären und der linksventrikulären Elektrode zu bewerten und immer dann eine rechts- bzw. linksventrikuläre Dislokation zu signalisieren, wenn ein plötzlicher Frequenzanstieg an der rechts- bzw. linksventrikulären Elektrode wahrgenommen wird ohne dass zeitnah eine nennenswerte Rhythmusänderung an der links- bzw. rechtventrikulären Elektrode erfasst wird, so dass bei einer signalisierten Dislokation einer der ventrikulären Elektroden die Rhythmusinformationen der betroffen Elektrode für die Tachykardiedetektion ignoriert werden können.

Vorzugsweise ist die Tachykardie-Erkennungseinheit ausgebildet, auf ein Dislokationssignal der Dislokations-Erkennungseinheit hin ein von einer jeweils betroffenen Elektrode stammendes Signal für die Tachykardiedetektion zu ignorieren. Dies kann auch dadurch bewirkt werden, dass der Tachykardie-Erkennungseinheit ein von einer als disloziert erkannten Elektrode stammendes Signal gar nicht erst zugeführt wird.

Vorzugsweise verfügt das Herztherapiegerät über eine zusätzliche atriale Elektrode, wobei das Herztherapiegerät ausgebildet ist, vorstehend beschriebene Dislokationsprüfung durch die Dislokations-Erkennungseinheit nur dann durchzuführen, wenn an der atrialen Elektrode ein Vorhofflimmern (atriale Fibrillation, AF)wahrgenommen wird. Z.B. kann eine AF-Erkennungseinheit zum Detektieren atrialer Fibrillationen vorgesehen sein, die im Falle einer detektierten atrialen Fibrillation ein AF-Signal ausgibt, welches u.a. eine Deaktivierung der Dislokations-Erkennungseinheit bewirkt.

Die Dislokations-Erkennungseinheit ist vorzugsweise ausgebildet, folgende Kriterien oder deren Kombination zusätzlich zur linksventrikulären Dislokationserkennung heranzuziehen: eine maximal erlaubte Vorzeitigkeit einer linksventrikulären Kontraktion vor einer rechtsventrikulären Kontraktion, Stabilität der A-RV-Überleitungszeit, d.h. der Überleitungszeit vom Atrium zum rechten Ventrikel, ein Frequenzvergleich zwischen Atrium und linkem Ventrikel, die linksventrikuläre Stimulationsreizschwelle, die Morphologie der linksventrikulären R-Wellen vor einer jeweils vorläufig erfassten und zu bestätigenden Tachykardie, Amplituden, Impedanzen, Reizschwellen.

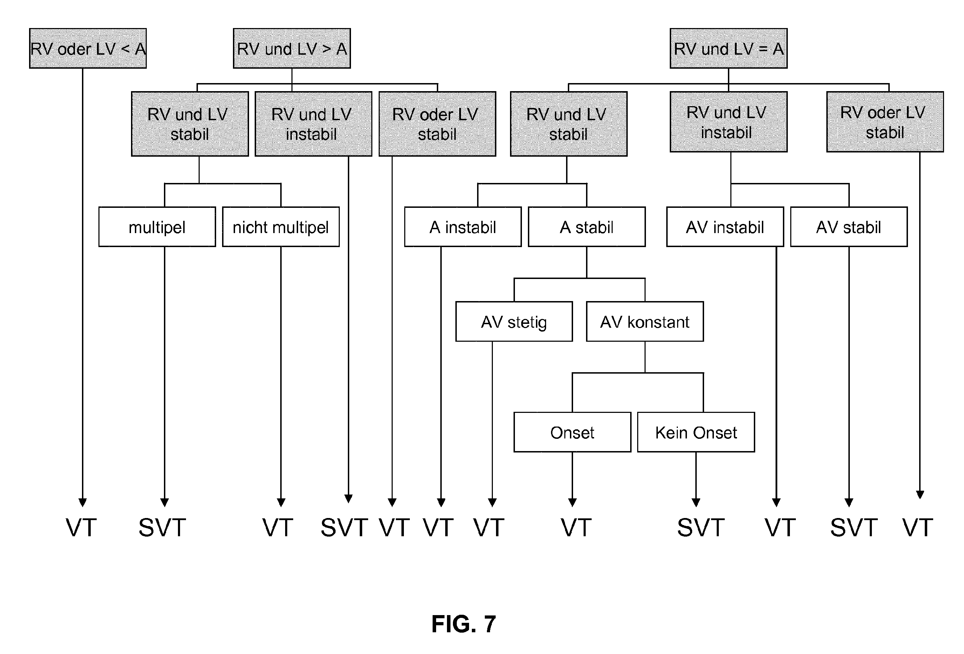

Vorzugsweise ist das Herztherapiegerät ein Dreikammer-Gerät, welches mit einer rechtsatrialen Elektrode, rechtsventrikulären Elektrode und einer linksventrikulären Elektrode verbunden oder zu verbinden ist. Die Tachykardie-Erkennungseinheit ist dann vorzugsweise so ausgebildet, dass sie anstelle von oder zusätzlich zu bekannten Zweikammer-Diskriminierungsalgorithmen (die das rechte Atrium und den rechten Ventrikel einbeziehen) einen demgegenüber erweiterten 3-Kammer-Diskriminierungsalgorithmen ausführt, bei denen zur Klassifikation des Ursprungs einer Tachykardie grundsätzlich auch die Intervallinformationen des linken Ventrikels aufgenommen werden und ebenso die A-LV-Überleitungszeiten vom Atrium zum linken Ventrikel berücksichtigt werden, beispielsweise wie dies in dem nachfolgend in Figur 7 dargestellten Ausführungsbeispiel illustriert ist.

Vorzugsweise ist die Tachykardie-Erkennungseinheit außerdem so ausgebildet, dass sie ein an sich bekanntes Onset-Kriterium (Sudden Onset, d.h. ein plötzlicher Anstieg der Herzrate innerhalb einer oder nur einer ganz geringen Anzahl von Herzzyklen) zur Diskriminierung zwischen einem physiologischem Frequenzanstieg (der zu einer Sinus-Tachykardie führt) und einer plötzlich einsetzenden ventrikulären Tachykardie um die linksventrikuläre Rhythmusbewertung erweitert und immer nur dann eine Sinus-Tachykardie signalsiert (indem sie ein entsprechend Tachyarrhythmiesignal ausgibt), wenn kein plötzlicher Frequenzanstieg im rechten Ventrikel erfolgt und auch kein plötzlicher Frequenzanstieg im linken Ventrikel erfolgt und/oder die interventrikulären Leitungszeiten bei Berücksichtigung einer Toleranz unverändert bleiben.

Vorzugsweise ist die Tachykardie-Erkennungseinheit außerdem so ausgebildet, dass sie ein an sich bekanntes ventrikuläres Stabilitätskriterium zur Unterscheidung zwischen einer stabilen monomorphen ventrikulären Tachykardie und einem übergeleitetem Vorhofflimmern in derart erweiterter Form anwendet, dass nur dann eine ventrikuläre Tachykardie (VT) detektiert wird, wenn der Rhythmus in beiden Ventrikeln als stabil klassifiziert wird.

Das Herztherapiegerät ist vorzugsweise dazu ausgebildet, die Zeitgebersteuerung durch die Therapiegerätsteuerung des Schrittmachers und damit insbesondere die für die Tachykardiedetektion erforderlichen Ausblendzeiten wie in US 2011/0082512 beschrieben immer dann automatisch auf die linksventrikuläre Seite umzuschalten (d.h. so dass die Zeiten ausgehend von erfassten linksventrikulären Ereignissen gesteuert werden), wenn die linksventrikulären Signale für die Tachykardiedetektion als geeignet klassifiziert werden, d.h. keine Dislokation erkannt wird. In Bezug auf eine weitere vorteilhafte Ausgestaltung eines solchen Therapiegerätes wird auf US 2011/0082512 bzw. EP 2 308 558 verwiesen, deren Offenbarung hierzu in diese Anmeldung mit einbezogen wird. Ausbelendzeit, also Zeiten, in denen eine jeweilige Sensingeinheit entweder kardiale Ereignisse nicht wahrnehmen kann oder Zeiten, zu denen wahrgenommene kardiale Ereignisse für die Tachykardieerkennung ignoriert werden, sind dem Fachmann grundsätzlich bekannt.

Vorzugsweise weist die Tachykardie-Erkennungseinheit für die biventrikuläre Detektion getrennte Detektionszähler für das rechtsventrikuläre und das linksventikuläre Signal auf und ist so ausgebildet, dass das Erreichen eines vorgegebenen Zählerstandes bereits eines der beiden Zähler eine entsprechende Detektion und damit ein entsprechendes Tachyarrhythmiesignal auslöst.

Alternativ weist die Tachykardie-Erkennungseinheit für die biventrikuläre Detektion gemeinsame Detektionszähler für das rechtsventrikuläre und das linksventrikuläre Signal auf, wobei diese bei abweichender Intervalldauer zwischen rechtsventrikulärem und das linksventrikulärem Signal immer von der schnelleren Ventrikelseite bestimmt werden.

In Bezug auf ein Terminierungskriterium, welches für die Therapiegerätsteuerung zum Beenden einer antitachykarden Therapie (ATP anti tachycardia pacing) heranzieht, ist es bevorzugt, wenn die Therapiegerätsteuerung ein Terminierungskriterium verwirklicht, welches nur dann als erfüllt gilt, wenn gemessene Intervalldauern sowohl im rechten Ventrikel als auch im linken Ventrikel größer als eine vorgegebene Intervallgrenze für die Terminierung sind.

Alternativ kann auch eine Therapiegerätsteuerung vorgesehen sein, die so ausgebildet ist, dass das Terminierungskriterium auch dann als erfüllt gilt, wenn die gemessene Intervalldauer nur im rechten Ventrikel größer als eine vorgegebene Intervallgrenze für die Terminierung ist.

Vorzugsweise verwirklicht das Herztherapiegerät auch eine rechts- und linksventrikuläre Undersense-Erkennung, z.B. mittels einer Plausibilitätsprüfung über rechts- und linksventrikuläre und optional atriale Intervallanzahlen, und ist ausgebildet immer dann auf eine rechtsventrikuläre oder linksventrikuläre Detektion umzuschalten, wenn eine der ventrikulären Elektroden ein deutliches Undersensing aufweist, d.h. wenn eine der ventrikulären Elektroden wegen teilweise nicht erfasster kardialer Ereignisse deutlich weniger Herzzyklen detektiert als, die andere oder anderen Elektroden.

Vorzugsweise ist die Tachykardie-Erkennungseinheit derart ausgebildet, dass sie zwischen einer rein rechtsventrikulären Tachykardieerkennung, bei der nur von einer rechtsventrikulären Elektrode und ggf. zusätzlich von einer atrialen Elektrode stammende Signale für die Tachykardieerkennung ausgewertet werden, und einer biventrikulären Tachykardieerkennung, bei der auch von einer linksventrikulären Elektrode stammende Signale für die Tachykardieerkennung ausgewertet werden, umschaltbar ist. So kann insbesondere nach einer Implantation des Herztherapiegerätes oder einer angeschlossenen Elektrodenleitung zunächst immer eine rein rechtsventrikuläre Detektion erfolgen, bis vom Herztherapiegerät eine stabile Elektrodenlage im linken Ventrikel automatisch festgestellt wurde und dann automatisch die biventrikuläre Detektion aktiviert wird. Vorzugsweise ist das Herztherapiegerät zu einer derartigen automatischen Umschaltung ausgebildet.

Das Herztherapiegerät bzw. dessen Therapiegerätsteuerung ist vorzugsweise derart ausgebildet, dass es bzw. sie nach jeder Abgabe eine Defibrillationsschocks eine zusätzliche Dislokationsprüfung der linksventrikulären Elektrode auf eine vorstehend erwähnte Weise durchführt.

Aus Vereinfachungsgründen ist die Dislokations-Erkennungseinheit vorzugsweise so ausgebildet, dass sie nur eine Dislokation einer linksventrikulären Elektrode erfasst, d.h. die von der Dislokations-Erkennungseinheit durchgeführte Dislokationserkennung bezieht sich nur auf eine jeweilige linksventrikuläre Coronar-Sinus-Elektrode, da hier die Wahrscheinlichkeit einer Dislokation größer ist.

Die Erfindung soll nun anhand eines Ausführungsbeispiels mit Bezug auf die Figuren näher erläutert werden.

Von den Figuren zeigt.

Figur 1:　ein Beispiel für eine dissimilare ventrikuläre Tachyarrhythmie

Figur 2:　einen biventrikulären Herzschrittmacher mit rechtsventrikulärer Defbrillationsschockwendel als implantierbaren Herzstimulator;

Figur 3:     einige Bestandteile des Herzstimulators auf Figur in Form eines vereinfachten Blockdiagramms;

Figur 4:     einen biventrikulären Dreikammer-Herzschrittmacher und implantierbaren Kardioverter/Defibrillator (ICD) als implantierbaren Herzstimulator;

Figur 5:     ein Flussdiagramm zur Illustration der Dislokationserkennung;

Figur 6:     ein Beispiel einer biventrikulären Detektion; und

Figur 7:     einen 3-Kammer-Diskriminierungsalgorithmus

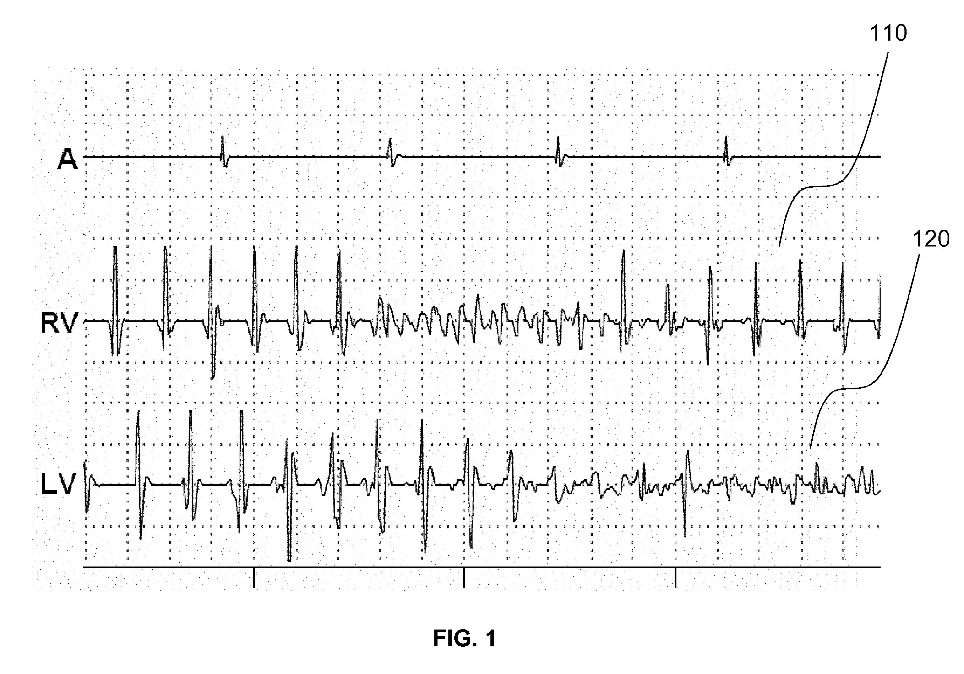

In der Figur 1 ist ein typisches Beispiel einer dissimilaren ventrikulären Tachyarrythmie dargestellt. Hier wechselt im rechten Ventrikel (RV) der Rhythmus von einer stabilen VT über eine kurze Phase von VF auf eine langsamere VT (110), gleichzeitig wechselt zu einem späteren Zeitpunkt der Rhythmus im LV-Kanal von einer stabilen VT zu einem dauerhaften VF, dass bei einer rein rechtsventrikulären Detektion nicht wahrgenommen wird und zu einer fehlerhaften Therapieauswahl führt.

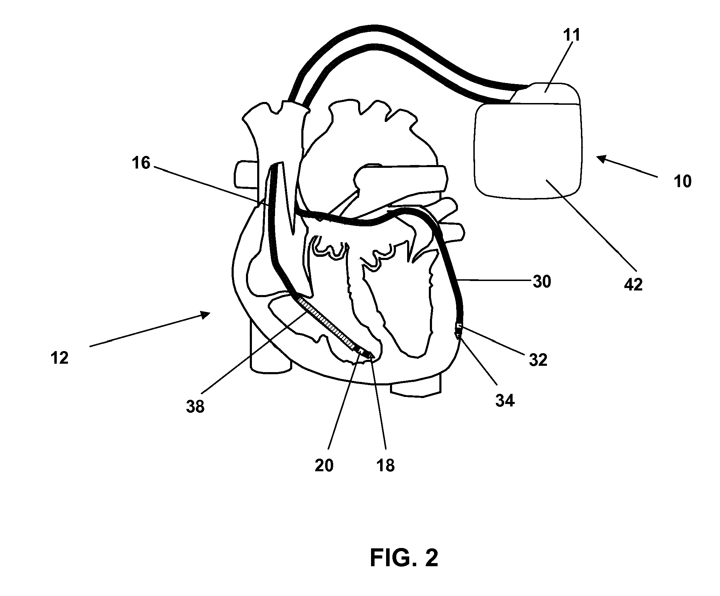

Figur 2 zeigt einen biventrikulären Herzschrittmacher/Defibrillator (ICD oder CRT-D) mit einer rechtsventrkulären Defbrillationsschockwendel als implantierbares Herztherapiegerät 10. Dieser biventrikuläre Herzschrittmacher ist über Elektrodenleitungen 16 und 30 mit Stimulations- und Sensingelektroden 18 und 20 bzw. 32 und 34 im rechten bzw. linken Ventrikel eines Herzens verbunden und kann auf diese Weise Stimulationsimpulse an das Herz abgeben und elektrische Potentiale vom Herzen aufnehmen.

Die Elektrodenleitungen 16 und 30 sind über bekannte, standarsierte Steckverbindungen mit Kontaktbuchsen in einem Header (Anschlussgehäuse) 11 des Herzstimulators 10 elektrisch verbunden. Auf diese Weise sind die Elektrodenleitungen 16 und 30 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 16 ist eine rechtsventrikuläre Elektrodenleitung und besitzt an ihrem distalen Ende einen rechtsventrikulären Spitzenelektrodenpol RV Tip 18 und in unmittelbarer Nähe einen rechtsventrikulären Ringelektrodenpol RV Ring 20. Beide Elektrodenpole sind im Apex des rechten Ventrikels des Herzens angeordnet.

Die Elektrodenleitung 30 ist eine linksventrikuläre Elektrodenleitung und weist am distalen Ende eine bipolare Stimulations- und Wahrnehmungselektrode mit einem distalen Spitzenelektrodenpol LV Tip 34 sowie in deren Nähe einem linksventrikulären Ringelektrodenpol LV Ring 32 auf. Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

Außerdem trägt die rechtsventrikuläre Elektrodenleitung 16 noch eine rechtsventrikuläre Schockwendel RV Schock 38 als großflächigen Elektrodenpol zur Abgabe von Defibrillationsschocks.

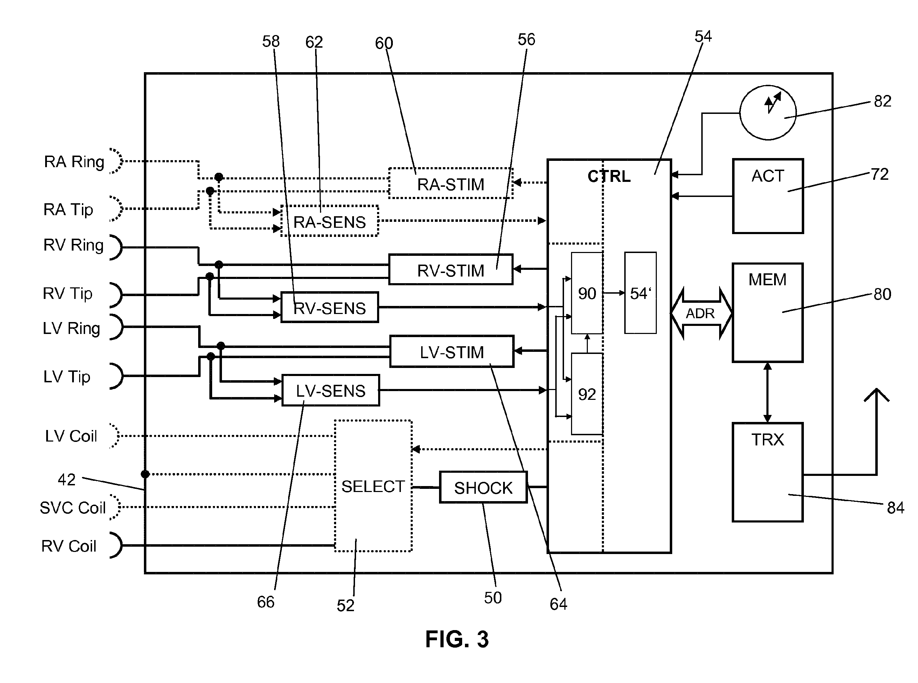

In Figur 3 sind einige der wesentlichen Funktionseinheiten des Herzstimulators 10 dargestellt. In punktierter Liniendarstellung sind außerdem weitere Komponenten dargestellt, wie sie bei einer alternativen Ausführungsvariante eines implantierbaren Herzstimulators zusätzlich vorgesehen sein können.

Auf der linken Seite sind die elektrischen Anschlüsse für die verschiedenen Elektrodenpole 18, 20, 32, 34, und 38 dargestellt. Die Schockelektrode (Schockwendel) 38 ist mit einem Schockimpulsgenerator 50 verbunden. Der Schockimpulsgenerator 50 ist mit einer Steuereinheit 54 verbunden, die den Schockimpulsgenerator 50 bei Bedarf zur Erzeugung und Abgabe eines Kardioversions- oder Defibrillationsschocks ansteuert. In diesem Sinne wirkt die Steuereinheit 54 als Therapiegerätsteuerung 54'. Die Therapiegerätsteuerung 54' ist nur andeutungsweise in Figur 3 eingezeichnet und ist beispielsweise mit dem Schockimpulsgenerator 50 sowie einer rechtsventrikulären Stimulationseinheit 56 und mit einer linksventrikulären Stimulationseinheit 64 verbunden.

Die Steuereinheit 54 umfasst eine Tachykardie-Erkennungseinheit 90 und eine Dislokations-Erkennungseinheit 92.

Der Anschluss für den rechtsventrikulären Spitzenelektrodenpol RV Tip sowie der Anschluss für den rechtsventrikulären Ringelektrodenpol RV Ring sind jeweils sowohl mit der rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Steuereinheit 54 verbunden.

Die rechtsventrikuläre Stimulationseinheit 56 ist dazu ausgebildet, auf ein Ansteuersignal der Steuereinheit 54 hin einen rechtsventrikulären Stimulationsimpuls zu erzeugen und über die Anschlüsse für den rechtsventrikulären Ringelektrodenpol und den rechtsventrikulären Spitzenelektrodenpol abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für den rechtsventrikulären Spitzenelektrodenpol RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung des rechtsventrikulären Spitzenelektrodenpol 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für den rechtsventrikulären Ringelektrodenpol RV Ring und den rechtsventrikulären Spitzenelektrodenpol RV Tip anliegende elektrische Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal (z.B. ein Markersignal) an die Steuereinheit 54 und deren Tachykardie-Erkennungseinheit 90 und Dislokations-Erkennungseinheit 92 aus.

In analoger Weise sind auch der Anschluss für den linksventrikulären Spitzenelektrodenpol LV Tip und der Anschluss für den linksventrikulären Ringelektrodenpol LV Ring mit der linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Steuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Aktivitätssensor 72 mit der Steuereinheit 54 verbunden. Der Aktivitätssensor 72 ist dazu ausgebildet, ein von der körperlichen Aktivität eines Patienten abhängiges Signal, zum Beispiel ein Bewegungssignal, zu erfassen und ein entsprechendes, die körperliche Aktivität des Patienten anzeigendes Signal an die Steuereinheit 54 auszugeben. Dies erlaubt es, dass die Steuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Steuereinheit 54 verbunden ist und es erlaubt, von der Steuereinheit 54 erzeugte oder ausgewertete Signale zu speichern. Andererseits erlaubt es die Speichereinheit 80, Steuerprogramme für die Steuereinheit 54 in veränderbarer Form zu speichern. Des Weiteren ist die Steuereinheit 54 mit einem Zeitgeber 84 verbunden.

Der Herzstimulator 10 verfügt über mindestens eine bidirektionale Telemetrieschnittstelle 84, um gespeicherte Daten vom elektronischen Implantat an ein externes Gerät 100 übertragen zu können und umgekehrt auch Programmiereinstellungen und Therapiekommandos von diesem externen Gerät 100 empfangen zu können.

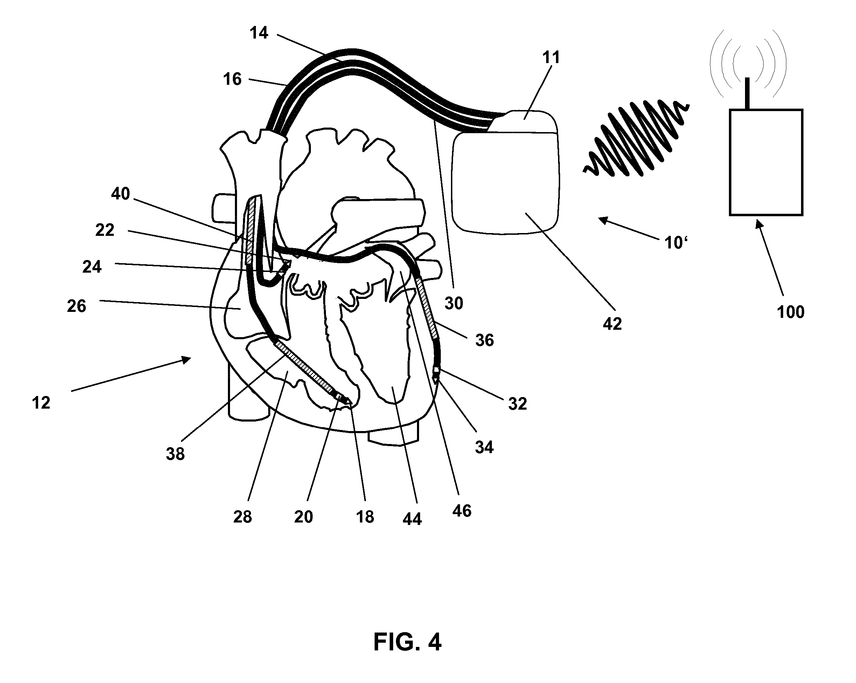

In Figur 4 ist eine alternative Ausführungsvariante eines implantierbaren Herztherapiegerätes in Form eines bi-ventrikulären Dreikammer-Herzstimulators / ICDs 10' dargestellt. Dieser ist über seinen Anschlussblock 11 (Header) mit einer rechtsventrikulären Elektrodenleitung 16, einer linksventrikulären Elektrodenleitung 30 und zusätzlich einer rechtsatrialen Elektrodenleitung 14 verbunden.

Diese Elektrodenleitungen werden im Herzen 12 dauerhaft implantiert. Die rechtsventrikuläre Elektrodenleitung 16 weist dabei am distalen Ende eine bipolare Stimulations- und Wahrnehmungselektrode mit Spitzenelektrodenpol RV Tip 18 und Ringelektrodenpol RV Ring 20 auf. Ferner ist diese Elektrodenleitung mit einer distalen Schockwendel RV Coil 38 und zusätzlich einer proximalen Schockwendel SVC Coil 40 ausgestattet. Die distale Schockwendel RV Coil 38 ist dabei so angeordnet, dass diese im rechten Ventrikel 28 liegt. Die proximale Schockwendel SVC Coil 40 liegt im oberen Teil des rechten Atriums 26 bzw. in der Superior vena cava (oberen Hohlvene).

Die Elektrodenleitung 14 ist eine atriale Elektrodenleitung weist am distalen Ende eine bipolare Stimulations- und Wahrnehmungselektrode, gebildet von einem Spitzenelektrodenpol RA Tip 22 und einem Ringelektrodenpol RA Ring 24 auf, implantiert im rechten Atrium 26.

Bei dem Ausführungsbeispiel in Figur 4 trägt die linksventrikuläre Elektrodenleitung 30 zusätzlich eine linksventrikuläre Schockwendel 36 zur Abgabe von Defibrillationsschocks an den linken Ventrikel. Diese Schockwendel 36 ist dabei so angeordnet, dass diese vom linken Ventrikel 44 bis hinauf zum linken Vorhof 46 reicht. Eine weitere Elektrode für die Schockabgabe stellt das elektrisch aktive Gehäuse 42 des Implantates 10 dar.

Wie Figur 3 anhand der punktiert dargestellten Komponenten zu entnehmen ist, sind der Anschluss für den rechtsatrialen Spitzenelektrodenpol und der Anschluss für den rechtsatrialen Ringelektrodenpol sowohl mit einer rechtsatrialen Stimulationseinheit 60 als auch mit einer rechtsatrialen Sensingeinheit 62 verbunden, die jeweils ihrerseits wiederum mit der Steuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Steuereinheit 54 weiter, welches eine natürliche Kontraktion des rechten Atriums kennzeichnet.

Die bei der in Figur 4 dargestellten Ausführungsvariante vorgesehene linksventrikuläre Schockwendel 36 ist - wie in Figur 3 ebenfalls punktiert dargestellt - über einen Anschluss LV-COIL und eine Elektrodenauswahleinheit 52 ebenfalls mit dem Schockgenerator 50 verbunden. Mittels der Elektrodenauswahleinheit 52 kann die Steuereinheit 54 zwei oder mehr Elektroden (einschließlich des leitenden Gehäuses 42) auswählen, über die ein Schock abgegeben werden soll.

Bei den in Figuren 2 bis 4 dargestellten Herztherapiegeräten erfolgt die Klassifikation der tachykarden ventrikulären Rhythmusstörungen primär über die wahrgenommenen Herzaktionen von der rechtsventrikulären und der linksventrikulären Elektrodenleitung simultan, wobei die schnellere Rhythmusstörung primär die Therapieauswahl bestimmt. Gleichzeitig erfolgt ebenso eine Prüfung auf eine mögliche Dislokation einer der ventrikulären Elektroden, um eine inadäquate Therapieabgabe zu verhindern. Wird eine solche Dislokation festgestellt, wird die betroffene Elektrode nicht mehr für die Tachykardiedetektion verwendet.

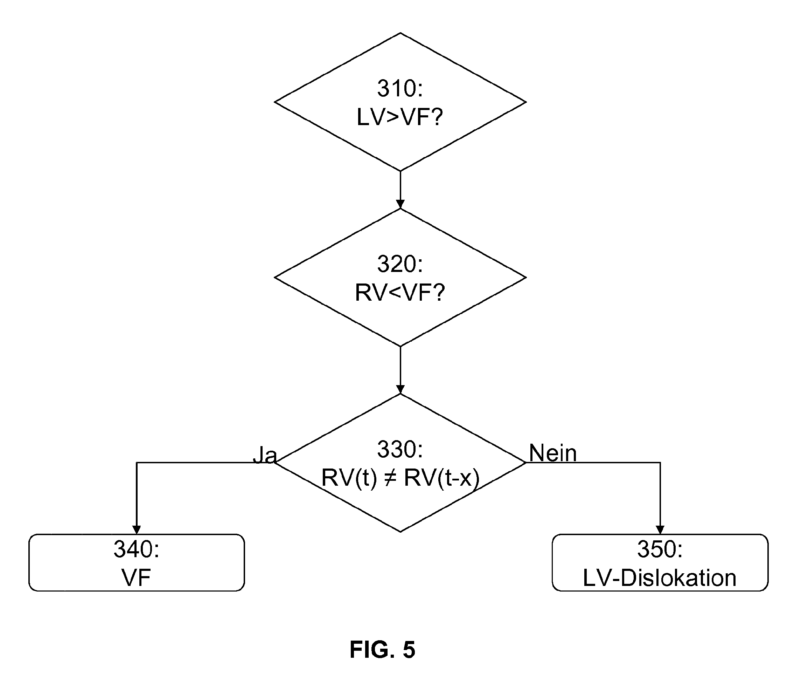

In Figur 5 ist die für die biventrikuläre Detektion vorgesehene und von der Dislokations-Erkennungseinheit 92 durchgeführte Dislokationserkennung am Beispiel einer LV-Dislokationserkennung, also einer Erkennung einer Dislokation der linksventrikulären Elektrode, in einem Flussdiagramm dargestellt.

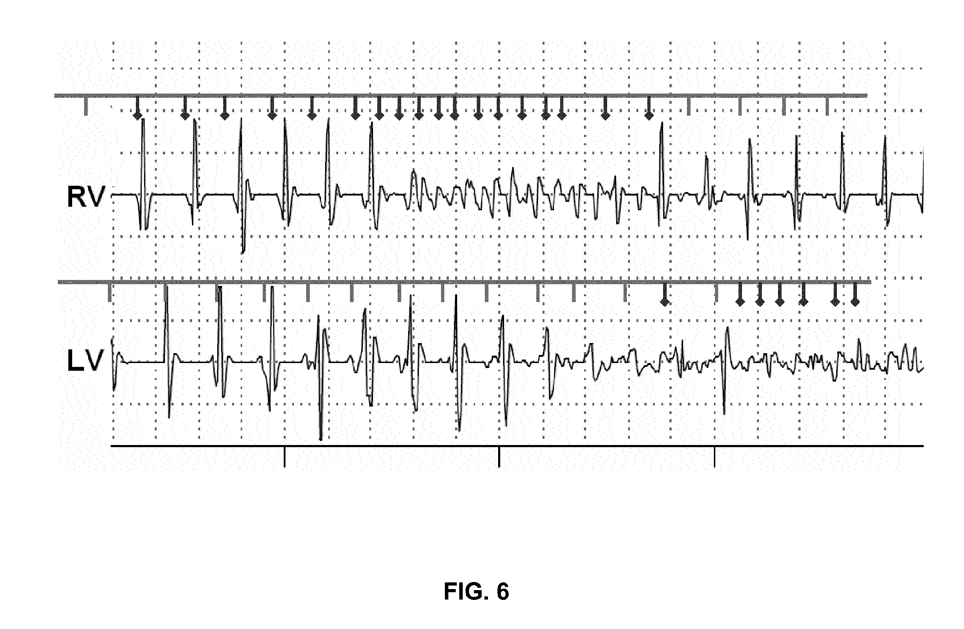

Da die linksventrikuläre Elektrodenleitung 30 (und damit die linksventrikuläre Elektrode, die den linksventrikulären Spitzenelektrodenpol LV Tip 34 und den linksventrikulären Ringelektrodenpol LV Ring 32 einschließt) innerhalb der Koronarvene derart verrutschen kann, dass deren Elektrodenpole 32 und 34 im Bereich des Atriums liegen, ist es nicht ausgeschlossen, dass eine vorhandene Vorhoftachykardie fälschlicher Weise als eine linksventrikuläre Tachykardie wahrgenommen wird und eine inadäquate Therapie bei biventrikulärer Detektion (siehe Figur 6) ausgelöst wird.

Um dies zu verhindern, prüft die Dislokations-Erkennungseinheit 92 zur Dislokationserkennung eine mögliche Dislokation der linksventrikulären Elektrode wie folgt:

Liegt die linksventrikuläre Rate in einem Bereich einer VT/VF-Zone (310) und liegt die rechtsventrikuläre Rate in keiner oder einer langsameren Zone (320), so wird überprüft, ob sich die rechtsventrikuläre Rate bei Beginn einer jeweiligen linksventrikulären Tachykardie signifikant geändert hat (330). Bleibt die rechtsventrikuläre Rate weitgehend unverändert, so detektiert das Herztherapiegerät eine Dislokation der linksventrikulären Elektrode (350), andernfalls eine tatsächliche ventrikuläre Arrhythmie (340).

Zur weiteren Verbesserung der Spezifität der Dislokationserkennung können optional weitere Elektroden und EKG-Ableitungen, wie eine rechtsatriale Elektrode oder ein Far-Field-EKG, eingesetzt werden. Die Kriterien zur LV-Dislokationserkennung können dabei z.B. folgende Informationen zusätzlich einschließen:

- Maximale Vorzeitigkeit eines LV-Sense vor RV-Sense
- Stabilitätsprüfung der A-RV-Überleitungszeit
- Vergleich der atrialen Frequenz mit der LV-Frequenz bzw. Intervalldauer
- LV-Stimulationsreizschwelle
- LV-R-Wellenmorphologieanalyse
- QRS-Far-Field-Analyse (bleibt die FF-QRS-Morphologie gleich, ist von einer Dislokation auszugehen, wenn L-VF angezeigt wird - spez. bei Vorhofflimmern)

In Figur 6 ist eine für eine biventrikuläre Detektion geeignete Zählerlogik dargestellt und am Beispiel von Abbildung 1 als Markerkette illustriert.

Die Detektion durch die Tachykardieerkennungseinheit erfolgt hier über nur einen Detektionszähler, der immer dann hochgezählt wird, wenn ein Intervall die programmierte Tachykardiezonengrenze unterschreitet. Als Intervalle werden dabei grundsätzlich rechts- und linksventrikulär wahrgenommene Intervalle zugelassen, wobei immer nur dann ein rechtsventrikuläres Intervall für die Zählung zugelassen wird, wenn dieses kürzer oder gleich dem vorangegangenen linksventrikulären Intervall ist. Ein linksventrikuläres Intervall wird immer nur dann für die Zählung zugelassen, wenn dieses kürzer als das vorangegangene rechtsventrikuläre Intervall ist.

Ein Detektionszähler der vorgenannten Art ist im nachfolgenden Beispiel durch die Funktion cnt(RV) realisiert, die einen Zählerwert immer dann um 1 hochzählt, wenn sie angesprochen wird:

$$IF\ RV(n) \leq LV(n\text{-}1)\ THEN\ cnt(RV);$$

$$IF\ LV(n) < RV(n\text{-}1)\ THEN\ cnt(RV);$$

So wird erreicht, dass immer nur die "schnellere" Ventrikelseite für die TachykardieBewertung herangezogen wird. In der Abbildung sind die für die Tachykardiebewertung zulässigen Intervallmarker mit folgendem Symbol gekennzeichnet:

In Figur 7 ist eine mögliche Variante eines 3-Kammerdiskriminierungsalgorithmus mit biventrikulärer Detektion beispielhaft dargestellt. Dieser demonstriert lediglich eine der möglichen Implementierungsvarianten, da sich die biventrikuläre Diskriminierung grundsätzlich in alle existierenden Diskriminierungsalgorithmen integrieren lässt und in jedem Fall eine Verbesserung der Sensitivität und Spezifität der VT/SVT-Diskriminierung (also der Unterscheidung originär ventrikulärer Tachykardien (VT) von supraventrikulären Tachykardien (SVT)) erwarten lässt.

Folgende Symbolik ist in Figur 7 verwendet:

RV:   Intervalldauer, gemessen an der rechtsventrikulären Elektrode

LV:   Intervalldauer, gemessen an der linksventrikulären Elektrode

A:   Intervalldauer, gemessen an der atrialen Elektrode

AV:   Atrio-ventrikuläre Leitungszeit (hier kann der Algorithmus durch eine Unterscheidung der rechts- und linksventrikulären Überleitung noch erweitert werden)

VT:   Bewertung der aktuellen Ventrikelerregung als Tachykardie ventrikulären Ursprungs

SVT:   Bewertung der aktuellen Ventrikelerregung als Tachykardie supraventrikulären Ursprungs

**Patentansprüche**

1.   Implantierbares biventrikuläres Herztherapiegerät (10) mit einer Therapiegerätsteuerung (54), die eine Tachykardie-Erkennungseinheit (90) aufweist, welche wenigstens indirekt mit einer rechtsventrikulären Wahrnehmungselektrode (18, 20) und einer linksventrikulären Wahrnehmungselektrode (32, 34) derart verbunden oder zu verbinden ist, dass der Tachykardie-Erkennungseinheit (90) im Betrieb einen zeitlichen Verlauf elektrischer Potentiale im Herzen repräsentierende Signale oder daraus abgeleitete Signale zugeführt werden, wobei die Tachykardie-Erkennungseinheit (90) ausgebildet ist, das ihr zugeführte Signal und dessen zeitlichen Verlauf auszuwerten und ein Tachyarrhythmiesignal zu generieren, falls das zugeführte Signal vorgegebene Kriterien erfüllt,
**dadurch gekennzeichnet, dass** die Tachykardie-Erkennungseinheit (90) ausgebildet ist, für die Erkennung von ventrikulären Tachykardien simultan die Herzfrequenz an der rechtsventrikulären und an der linksventrikulären Elektrode zu bewerten, und dass die Therapiegerätsteuerung (54) außerdem eine Dislokations-Erkennungseinheit (92) aufweist, die ebenfalls wenigstens indirekt mit einer rechtsventrikulären Wahrnehmungselektrode (18, 20) und einer linksventrikulären Wahrnehmungselektrode (32, 34) derart verbunden oder zu verbinden ist, dass der Dislokations-Erkennungseinheit (92) im Betrieb einen zeitlichen Verlauf elektrischer Potentiale im Herzen repräsentierende Signale oder daraus abgeleitete Signale zugeführt werden, wobei die Dislokations-Erkennungseinheit (92) ausgebildet ist, simultan die Herzfrequenz an der rechtsventrikulären und der linksventrikulären Elektrode (18, 20; 32, 34) zu bewerten und immer dann eine rechts- bzw. linksventrikuläre Dislokation zu signalisieren und ein entsprechendes Dislokationssignal zu generieren, wenn die Dislokations-Erkennungseinheit (92) einen plötzlichen Frequenzanstieg an der rechts- bzw. linksventrikulären Elektrode (18, 20; 32, 34) wahrnimmt, ohne dass innerhalb eines vorgegebenen und/oder einstellbaren Zeitfensters eine nennenswerte Rhythmusänderung an der links- oder der rechtventrikulären Elektrode erfasst wird, wobei die Therapiegerätsteuerung (54) weiterhin ausgebildet ist, bei einer signalisierten Dislokation der rechtsventrikulären oder der linksventrikulären Elektrode die Rhythmusinformationen der betroffenen Elektrode für die Tachykardiedetektion zu ignorieren.

2.   Herztherapiegerät nach Anspruch 1, bei dem die Tachykardie-Erkennungseinheit (90) ausgebildet ist, auf ein Dislokationssignal der Dislokations-Erkennungseinheit (92) hin ein von einer jeweils betroffenen Elektrode stammendes Signal für die Tachykardiedetektion zu ignorieren.

3.   Herztherapiegerät nach Anspruch 1 oder 2, wobei das Herztherapiegerät (10) wenigstens indirekt mit einer atrialen Wahrnehmungselektrode (22, 24) wirkverbunden oder zu verbinden und ausgebildet ist, eine Dislokationsprüfung durch die Dislokations-Erkennungseinheit (92) nur dann durchzuführen, wenn das Herztherapiegerät (10) über die atriale Wahrnehmungselektrode (22, 24) ein Vorhofflimmern wahrnimmt.

4.   Herztherapiegerät nach einem der Ansprüche 1 bis 3, bei dem die Dislokations-Erkennungseinheit (92) ausgebildet ist, eines oder mehrere der folgenden Kriterien für eine linksventrikuläre Dislokationserkennung zusätzlich heranzuziehen: eine maximal erlaubte Vorzeitigkeit einer linksventrikulären Kontraktion vor einer rechtsventrikulären Kontraktion, Stabilität der A-RV-Überleitungszeit, einen Frequenzvergleich zwischen Atrium und linkem Ventrikel, eine linksventrikuläre Stimulationsreizschwelle, die Morphologie der linksventrikulären R-Wellen vor einer jeweils vorläufig erfassten und zu bestätigenden Tachykardie, Amplituden, Impedanzen, Reizschwellen.

5. Herztherapiegerät nach einem der Ansprüche 1 bis 4, wobei das Herztherapiegerät (10) ein Dreikammer-Gerät (10') ist, welches mit einer rechtsatrialen Elektrode (22, 24), rechtsventrikulären Elektrode (18, 20) und einer linksventrikulären Elektrode (32, 34) wirkverbunden oder zu verbinden ist.

6. Herztherapiegerät nach Anspruch 5, bei dem die Tachykardie-Erkennungseinheit (90) derart ausgebildet ist, dass sie anstelle von oder zusätzlich zu Zweikammer-Diskriminierungsalgorithmen, welche vom rechten Atrium und vom rechten Ventrikel stammende Signale verarbeiten, einen demgegenüber erweiterten 3-Kammer-Diskriminierungsalgorithmus ausführt, bei dem zur Klassifikation des Ursprungs einer Tachykardie auch Intervallinformationen des linken Ventrikels aufgenommen und ebenso A-LV-Überleitungszeiten vom Atrium zum linken Ventrikel berücksichtigt werden.

7. Herztherapiegerät nach einem der Ansprüche 1 bis 6, bei dem die Tachykardie-Erkennungseinheit (90) derart ausgebildet ist, dass sie ein Sudden-Onset-Kriterium zur Diskriminierung zwischen einem physiologischem Frequenzanstieg und einer plötzlich einsetzenden ventrikulären Tachykardie um die linksventrikuläre Rhythmusbewertung erweitert und immer nur dann eine Sinus-Tachykardie signalisiert, wenn kein plötzlicher Frequenzanstieg im rechten Ventrikel erfolgt und auch kein plötzlicher Frequenzanstieg im linken Ventrikel erfolgt und/oder die interventrikulären Leitungszeiten bei Berücksichtigung einer Toleranz unverändert bleiben.

8. Herztherapiegerät nach einem der Ansprüche 1 bis 7, bei dem die Tachykardie-Erkennungseinheit (90) derart ausgebildet ist, dass sie ein ventrikuläres Stabilitätskriterium zur Unterscheidung zwischen einer stabilen monomorphen ventrikulären Tachykardie und einem übergeleitetem Vorhofflimmern in derart erweiterter Form anwendet, dass nur dann eine ventrikuläre Tachykardie (VT) detektiert wird, wenn der Rhythmus in beiden Ventrikeln als stabil klassifiziert wird.

9. Herztherapiegerät nach einem der Ansprüche 1 bis 8, wobei das Herztherapiegerät (10) dazu ausgebildet ist, die Zeitgebersteuerung durch die Therapiegerätsteuerung (54) des Herztherapiegerät (10) immer dann automatisch auf eine Steuerung basierend auf im linken Ventrikel erfasster Signale umzuschalten, wenn die linksventrikulären Signale für die Tachykardiedetektion als geeignet klassifiziert werden, und insbesondere keine linksventrikuläre Dislokation erkannt wird.

10. Herztherapiegerät nach einem der Ansprüche 1 bis 9, bei dem die Tachykardie-Erkennungseinheit (90) für die biventrikuläre Detektion getrennte Detektionszähler (cnt(RV)) für vom rechten und linken Ventrikel stammende Signale aufweist und derart ausgebildet ist, dass das Erreichen eines vorgegebenen Zählerstandes bereits eines der beiden Zähler eine entsprechende Detektion und damit ein entsprechendes Tachyarrhythmiesignal auslöst.

11. Herztherapiegerät nach einem der Ansprüche 1 bis 9, bei dem die Tachykardie-Erkennungseinheit (90) für die biventrikuläre Detektion gemeinsame Detektionszähler (cnt(RV)) vom rechten und linken Ventrikel stammende Signale aufweist, wobei die Tachykardiedetektion bei abweichender Intervalldauer zwischen rechtsventrikulärem und das linksventrikulärem Signal immer von der schnelleren Ventrikelseite bestimmt wird.

12. Herztherapiegerät nach einem der Ansprüche 1 bis 11, bei dem die Tachykardie-Erkennungseinheit (90) derart ausgebildet ist, dass sie zwischen einer rein rechtsventrikulären Tachykardieerkennung und einer biventrikulären Tachykardieerkennung umschaltbar ist.

13. Herztherapiegerät nach Anspruch 5, bei dem die Tachykardie-Erkennungseinheit (90) derart ausgebildet ist, dass sie nach einer Implantation des Herztherapiegerätes (10) oder einer angeschlossenen Elektrodenleitung (14; 16; 30) zunächst immer eine rein rechtsventrikuläre Detektion durchführt, bis vom Herztherapiegerät (10) eine stabile Elektrodenlage im linken Ventrikel automatisch festgestellt wurde und dann automatisch die biventrikuläre Detektion aktiviert.

14. Herztherapiegerät nach einem der Ansprüche 1 bis 13, bei dem die Therapiegerätsteuerung (54) derart ausgebildet ist, dass sie nach jeder Abgabe eine Defibrillationsschocks eine zusätzliche Dislokationsprüfung der linksventrikulären Elektrode durchführt..

15. Herztherapiegerät nach einem der Ansprüche 1 bis 14, bei dem die Dislokations-Erkennungseinheit (92) derart ausgebildet ist, dass sie ausschließlich eine Dislokation einer linksventrikulären Elektrode (32, 34) erfassen kann.

## Claims

1. An implantable biventricular heart therapy device (10) having a therapy device controller (54), which has a tachycardia identification unit (90), which is connected or is to be connected at least indirectly to a right-ventricular sensing electrode (18, 20) and a left-ventricular sensing electrode (32, 34) in such a way that, during operation, signals representing a course over time of electrical potentials in the heart or signals derived therefrom are fed to the tachycardia identification unit (90), wherein the tachycardia identification unit (90) is configured to evaluate the signal fed thereto and the course over time thereof and to generate a tachyarrhythmia signal if the fed signal meets predefined criteria,

**characterised in that** the tachycardia identification unit (90) is configured, for the identification of ventricular tachycardias, to simultaneously evaluate the heart rate at the right-ventricular and at the left-ventricular electrode,

and **in that** the therapy device controller (54) additionally has a dislocation identification unit (92), which likewise is connected or is to be connected at least indirectly to a right-ventricular sensing electrode (18, 20) and to a left-ventricular sensing electrode (32, 34) in such a way that, during operation, signals representing a course over time of electrical potentials in the heart or signals derived therefrom are fed to the dislocation identification unit (92), wherein the dislocation identification unit (92) is configured to simultaneously evaluate the heart rate at the right-ventricular and the left-ventricular electrode (18, 20; 32, 34) and to then signal a right-ventricular or left-ventricular dislocation and to generate a corresponding dislocation signal whenever the dislocation identification unit (92) senses a sudden rise in heart rate at the right-ventricular or left-ventricular electrode (18, 20; 32, 34) without detecting a significant rhythm change at the left-ventricular or the right-ventricular electrode within a predefined and/or adjustable time window,

wherein the therapy device controller (54) is also configured such that, in the event of a signalled dislocation of the right-ventricular or of the left-ventricular electrode, the rhythm information of the electrode in question is ignored for the tachycardia detection.

2. The heart therapy device according to Claim 1, in which the tachycardia identification unit (90) is configured such that, following a dislocation signal of the dislocation identification unit (92), a signal originating from an electrode concerned in each case is ignored for the tachycardia detection.

3. The heart therapy device according to Claim 1 or 2, wherein the heart therapy device (10) is operatively connected or is to be connected at least indirectly to an atrial sensing electrode (22, 24) and is configured to only carry out a dislocation check by means of the dislocation identification unit (92) when the heart therapy device (10) senses an atrial fibrillation via the atrial sensing electrode (22, 24).

4. The heart therapy device according to one of Claims 1 to 3, in which the dislocation identification unit (92) is configured to additionally use one or more of the following criteria for a left-ventricular dislocation identification: a maximum permissible anteriority of a left-ventricular contraction before a right-ventricular contraction, stability of the A-RV conduction time, a rate comparison between the atrium and the left ventricle, a left-ventricular stimulation stimulus threshold, the morphology of the left-ventricular R-waves before a tachycardia detected preliminarily in each case and to be confirmed, amplitudes, impedances and stimulus thresholds.

5. The heart therapy device according to one of Claims 1 to 4, wherein the heart therapy device (10) is a three-chamber device (10'), which is operatively connected or is to be connected to a right-atrial electrode (22, 24), right-ventricular electrode (18, 20) and a left-ventricular electrode (32, 34).

6. The heart therapy device according to Claim 5, in which the tachycardia identification unit (90) is configured in such a way that, instead of or additionally to two-chamber discrimination algorithms, which process signals originating from the right atrium and from the right ventricle, it carries out an extended 3-chamber discrimination algorithm, in which interval information of the left ventricle is also recorded for classification of the origin of a tachycardia and A-LV conduction times from the atrium to the left ventricle are also taken into consideration.

7. The heart therapy device according to one of Claims 1 to 6, in which the tachycardia identification unit (90) is configured in such a way that, for discrimination between a physiological rise in heart rate and a suddenly occurring ventricular tachycardia, it extends a sudden-onset criterion by the left-ventricular rhythm evaluation and only signals a sinus tachycardia whenever there is no sudden rise in heart rate in the right ventricle and also no sudden rise in heart rate in the left ventricle and/or the interventricular line times remain unchanged under consideration of a tolerance.

8. The heart therapy device according to one of Claims 1 to 7, in which the tachycardia identification unit (90) is configured in such a way that it applies a ventricular stability criterion in order to distinguish between a stable monomorphic ventricular tachycardia and a conducted atrial fibrillation in a form extended in such a way that a ventricular tachycardia (VT) is then only detected when the rhythm in both ventricles is classified as stable.

9. The heart therapy device according to one of Claims 1 to 8, wherein the heart therapy device (10) is configured to automatically switch the timer controller by means of the therapy device controller (54) of the heart therapy device (10) to a control based on signals detected in the left ventricle whenever the left-ventricular signals are classified as suitable for the tachycardia detection, and in particular no left-ventricular dislocation is identified.

10. The heart therapy device according to one of Claims 1 to 9, in which the tachycardia identification unit (90) comprises separate detection counters (cnt(RV)) for signals originating from the right and left ventricle for the biventricular detection and is configured in such a way that, when a predefined counter state is reached, one of the two counters already triggers a corresponding detection and therefore a corresponding tachyarrhythmia signal.

11. The heart therapy device according to one of Claims 1 to 9, in which the tachycardia identification unit (90) comprises common detection counters (cnt(RV)) for signals originating from the right and left ventricle for the biventricular detection, wherein, in the event of a deviating interval time between the right-ventricular and the left-ventricular signal, the tachycardia detection is always determined by the quicker ventricle side.

12. The heart therapy device according to one of Claims 1 to 11, in which the tachycardia identification unit (90) is configured in such a way that it can be switched over between a purely right-ventricular tachycardia identification and a biventricular tachycardia identification.

13. The heart therapy device according to Claim 5, in which the tachycardia identification unit (90) is configured in such a way that, after implantation of the heart therapy device (10) or a connected electrode line (14; 16; 30), a purely right-ventricular detection is initially always carried out until a stable electrode position in the left ventricle has been automatically determined by the heart therapy device (10) and then automatically activates the biventricular detection.

14. The heart therapy device according to one of Claims 1 to 13, in which the therapy device controller (54) is configured in such a way that, after each delivery of a defibrillation shock, it carries out an additional dislocation check of the left-ventricular electrode.

15. The heart therapy device according to one of Claims 1 to 14, in which the dislocation identification unit (92) is configured in such a way that it can detect exclusively a dislocation of a left-ventricular electrode (32, 34).

**Revendications**

1. Appareil thérapeutique cardiaque (10) bi-ventriculaire pouvant être implanté, avec un système de commande de l'appareil thérapeutique (54) qui présente une unité de reconnaissance de tachycardie (90), laquelle est connectée, ou est à connecter, au moins indirectement avec une électrode de reconnaissance du ventricule droit (18, 20) et une électrode de reconnaissance du ventricule gauche (32, 34) de telle sorte que des signaux représentant un cours de temps des potentiels électriques dans le coeur, ou des signaux en étant la conséquence, sont transmis à l'unité de reconnaissance de tachycardie (90) en fonctionnement, où l'unité de reconnaissance de tachycardie (90) est conçue pour exploiter le signal qui lui est transmis et son évolution dans le temps, et pour générer un signal de tachyarythmie lorsque le signal transmis correspond à des critères prédéfinis **caractérisé en ce que** l'unité de reconnaissance de tachycardie (90) est conçue pour évaluer la fréquence cardiaque simultanément sur l'électrode ventriculaire droite et sur l'électrode ventriculaire gauche pour la reconnaissance de tachycardies ventriculaires, et que le système de commande de l'appareil thérapeutique (54) présente en outre une unité de reconnaissance de délocalisation (92) qui est également connectée, ou est à connecter, au moins indirectement avec une électrode de reconnaissance du ventricule droit (18, 20) et une électrode de reconnaissance du ventricule gauche (32, 34) de telle sorte que des signaux représentant un cours de temps des potentiels électriques dans le coeur, ou des signaux en étant la conséquence, sont transmis à l'unité de reconnaissance de délocalisation (92) en fonctionnement, où l'unité de reconnaissance de délocalisation (92) est conçue pour évaluer simultanément la fréquence cardiaque sur l'électrode ventriculaire droite et sur l'électrode ventriculaire gauche (18, 20 ; 32, 34) et pour signaler toujours ensuite une délocalisation dans le ventricule droit, respectivement, gauche, et générer un signal de délocalisation correspondant, lorsque l'unité de reconnaissance de délocalisation (92) perçoit une élévation brusque de la fréquence sur l'électrode ventriculaire droite, respectivement, gauche, sans qu'une modification de rythme significative sur l'électrode ventriculaire gauche ou droite ne soit décelée pendant un laps de temps prédéfini, et/ou réglable, où le système de commande de l'appareil thérapeutique (54) est en outre conçu pour ignorer les informations concernant le rythme de l'électrode concernée pour la détection de la tachycardie pour une délocalisation signalé de l'électrode ventriculaire droite ou de l'électrode ventriculaire gauche.

2. Appareil thérapeutique cardiaque selon la revendication 1, dans lequel l'unité de reconnaissance de tachycardie (90) est conçue pour ignorer le signal pour la détection de la tachycardie provenant de l'électrode concernée respective après un signal de délocalisation de l'unité de reconnaissance de délocalisation (92).

3. Appareil thérapeutique cardiaque selon les revendications 1 ou 2, où l'appareil thérapeutique cardiaque (10) est connecté fonctionnellement, ou est à connecter, au moins indirectement avec une électrode de reconnaissance de l'atrium (22, 24) et est conçu pour exécuter un examen de délocalisation par l'unité de reconnaissance de délocalisation (92) uniquement si l'appareil thérapeutique cardiaque (10) perçoit une fibrillation auriculaire par l'électrode de reconnaissance de l'atrium (22, 24).

4. Appareil thérapeutique cardiaque selon l'une des revendications 1 à 3, dans lequel l'unité de reconnaissance de délocalisation (92) est conçue pour mettre en évidence de manière complémentaire un ou de plusieurs parmi les critères suivants pour la reconnaissance d'une délocalisation ventriculaire gauche : une avance maximale permise d'une contraction ventriculaire gauche devant une contraction ventriculaire droite, la stabilité de la durée de dépolarisation A-VD, une comparaison de la fréquence entre l'atrium et le ventricule gauche, un seuil d'excitation par stimulation ventriculaire gauche, la morphologie des ondes R ventriculaires gauches avant une tachycardie provisoirement détectée et à confirmer, les amplitudes, les impédances, les seuils d'excitation.

5. Appareil thérapeutique cardiaque selon l'une des revendications 1 à 4, où l'appareil thérapeutique cardiaque (10) est un appareil à trois chambres (10'), lequel est connecté fonctionnellement, ou est à connecter, avec une électrode atriale droite (22, 24), une électrode ventriculaire droite (18, 20) et une électrode ventriculaire gauche (32, 34).

6. Appareil thérapeutique cardiaque selon la revendication 5, dans lequel l'unité de reconnaissance de tachycardie (90) est conçue de telle manière qu'à la place ou complémentairement aux algorithmes de discrimination de deux chambres, lesquels traitent les signaux provenant de l'atrium droit et du ventricule droit, elle présente en outre un algorithme de discrimination pour une troisième chambre, dans laquelle aussie des informations concernant les intervalles du ventricule gauche sont enregistrées et également les durées des dépolarisation A-VG de l'atrium vers le ventricule gauche sont prises en compte pour la classification de l'origine d'une tachycardie.

7. Appareil thérapeutique cardiaque selon l'une des revendications 1 à 6, dans lequel l'unité de reconnaissance de tachycardie (90) est conçue de telle manière qu'elle élargit un critère d'apparition soudaine pour la discrimination entre une augmentation de fréquence physiologique et une tachycardie ventriculaire survenant soudainement à l'évaluation du rythme ventriculaire gauche et signale toujours une tachycardie sinusale uniquement lorsqu'aucune augmentation de fréquence brusque n'est réalisée dans le ventricule droit et également lorsqu'il n'y a pas d'augmentation de fréquence brusque dans le ventricule gauche et/ou lorsque les temps de conduction interventriculaires restent inchangés en tenant compte d'une tolérance.

8. Appareil thérapeutique cardiaque selon l'une des revendications 1 à 7, dans lequel l'unité de reconnaissance de tachycardie (90) est conçue de telle manière quelle emploie un critère de stabilité ventriculaire pour la différentiation entre une tachycardie ventriculaire monomorphe stable et une fibrillation auriculaire se superposant dans une forme élargie de telle sorte qu'une tachycardie ventriculaire (TV) est détectée uniquement lorsque le rythme dans les deux ventricules se situe dans une catégorie stable.

9. Appareil thérapeutique cardiaque selon l'une des revendications 1 à 8, où l'appareil thérapeutique cardiaque (10) est conçu pour changer le réglage du système de commande de l'horloge par le système de commande de l'appareil thérapeutique (54) de l'appareil thérapeutique cardiaque (10) toujours automatiquement vers un système de commande basé sur des signaux détectés sur le ventricule gauche lorsque les signaux ventriculaires gauches pour la détection de la tachycardie sont classés de manière appropriée et qu'en particulier aucune délocalisation ventriculaire gauche n'est reconnu.

10. Appareil thérapeutique cardiaque selon l'une des revendications 1 à 9, dans lequel l'unité de reconnaissance de tachycardie (90) présente des compteurs de détection (cnt(RV) séparés pour des signaux provenant du ventricule droit et du ventricule gauche dans le cas de la détection bi-ventriculaire, et est conçue de telle manière que, lorsqu'un état de comptage prédéfini est atteint déjà pour l'un des deux compteurs, une détection correspondante, et par conséquent, un signal de tachyarythmie correspondant, est déclenché.

11. Appareil thérapeutique cardiaque selon l'une des revendications 1 à 9, dans lequel l'unité de reconnaissance de tachycardie (90) présente des compteurs de détection (cnt(RV) communs pour des signaux provenant du ventricule droit et du ventricule gauche dans le cas de la détection bi-ventriculaire, où la détection de la tachycardie est évaluée toujours à partir du côté de ventricule le plus rapide lors d'une durée d'intervalle en déviation entre le signal ventriculaire droit et le signal ventriculaire gauche.

**12.** Appareil thérapeutique cardiaque selon l'une des revendications 1 à 11, dans lequel l'unité de reconnaissance de tachycardie (90) est conçue de telle manière qu'elle peut être changée pour le réglage entre une reconnaissance d'une tachycardie purement ventriculaire droite et une reconnaissance d'une tachycardie bi-ventriculaire.

**13.** Appareil thérapeutique cardiaque selon la revendication 5, dans lequel l'unité de reconnaissance de tachycardie (90) est conçue de telle manière qu'elle exécute toujours d'abord une détection purement ventriculaire à droite après une implantation de l'appareil thérapeutique cardiaque (10) ou d'une ligne d'électrode (14 ; 16 ; 30) connectée, jusqu'à ce qu'une position d'électrode stable dans le ventricule gauche soit détectée automatiquement et active ensuite automatiquement la détection bi-ventriculaire.

**14.** Appareil thérapeutique cardiaque selon l'une des revendications 1 à 13, dans lequel le système de commande de l'appareil thérapeutique (54) est conçu de telle manière qu'après chaque délivrance d'un choc de défibrillation, il exécute un examen complémentaire de délocalisation de l'électrode ventriculaire gauche.

**15.** Appareil thérapeutique cardiaque selon l'une des revendications 1 à 14, dans lequel l'unité de reconnaissance de délocalisation (92) est conçue de telle manière qu'elle peut détecter exclusivement une délocalisation d'une électrode ventriculaire gauche 132, 34).

FIG. 1

EP 2 826 524 B1

EP 2 826 524 B1

**FIG. 2**

**FIG. 3**

FIG. 4

EP 2 826 524 B1

```
        ┌─────────────┐
        │   310:      │
        │   LV>VF?    │
        └─────────────┘
               │
               ▼
        ┌─────────────┐
        │   320:      │
        │   RV<VF?    │
        └─────────────┘
               │
               ▼
Ja    ┌─────────────────┐    Nein
 ┌────│      330:        │────┐
 │    │  RV(t) ≠ RV(t-x) │    │
 │    └─────────────────┘    │
 ▼                            ▼
┌──────────┐          ┌────────────────┐
│  340:    │          │     350:       │
│  VF      │          │  LV-Dislokation│
└──────────┘          └────────────────┘
```

**FIG. 5**

RV

LV

FIG. 6

RV oder LV < A  | RV und LV > A | RV und LV = A

RV und LV stabil | RV und LV instabil | RV oder LV stabil | RV und LV stabil | RV und LV instabil | RV oder LV stabil

multipel | nicht multipel | A instabil | A stabil | AV instabil | AV stabil

AV stetig | AV konstant

Onset | Kein Onset

VT  SVT  VT  SVT  VT  VT  VT  VT  SVT  VT  SVT  VT

**FIG. 7**

EP 2 826 524 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110082512 A
- EP 2308558 A